# EUROPEAN PATENT SPECIFICATION

(11) **EP 0 760 649 B1**
(45) Date of publication and mention of the grant of the patent: **31.10.2001**
(21) Application number: 95920841.4
(22) Date of filing: 19.05.1995
(51) Int. Cl.: A61K 9/00, A61K 31/56

(54) **FLUTICASONE PROPIONATE FORMULATIONS**
FLUTICASONPROPIONAT-FORMULIERUNGEN
FORMULATIONS A BASE DE PROPIONATE DE FLUTICASONE

(30) Priority: 21.05.1994 GB 9410222
(43) Date of publication of application: 12.03.1997
(73) Proprietor: GLAXO WELLCOME AUSTRALIA LTD., Boronia, VIC 3155 (AU)
(72) Inventor: TAINSH, David, Alexander Glaxo Research and, Park Road, Ware Hertfordshire SG12 0DP (GB); ILOTT, Trevor, Leslie Glaxo Research and, Ware Hertfordshire SG12 0DP (GB); SNELL, Dorothy, Jill, Boronia, VIC 3155 (AU); LAM, Li, Fong, Boronia, VIC 3155 (AU)
(74) Representative: Hackett, Ruth Elizabeth
(86) International application number: PCT/EP95/01913
(87) International publication number: WO 95/31964

(56) References cited:
- WO-A-93/17665
- DRUG INVESTIGATION, vol.8, no.3, 1994 pages 127 - 133 AINGE, G.; ET AL. 'Lack of deleterious effects of corticosteroid sprays containing benzalkonium chloride on nasal ciliated epithelium'
- RHINOLOGY, vol.11, 1991 pages 37 - 43 SCADDING, G.K.; ET AL. 'clinical and physiological effects of fluticasone propionate aqueous nasal spray in the treatment of perennial rhinitis'

## Description

This invention relates to improvements in or relating to pharmaceutical compositions comprising a fluticasone ester. In particular the invention relates to novel formulations of use in the administration of fluticasone propionate by inhalation.

Fluticasone propionate is the approved name for S-fluoromethyl 6a, 9a-difluoro-11b-hydroxy-16a-methyl-17a-propionyloxy-3-oxandrosta-1,4-diene 17b carbothioate, a corticosteroid known to exhibit topical antiinflammatory activity and described and claimed in GB 2088877. In the treatment of asthmatic conditions it has been found to be effective to administer fluticasone propionate in the form of dry powders or aerosols containing small particles of the medicament, conventionally prepared by micronisation. Conventionally, fluticasone propionate aerosols have been administered by means of metered dose inhalers, which are designed to deliver a fixed unit dosage of medicament per actuation or "puff". However, some patients, in particular children and the elderly, have difficulty in co-ordinating actuation of a metered dose inhaler with inhalation, and are therefore unable to use this mode of administration effectively. Furthermore, a proportion of patients find inhalation of dry powders difficult or unpleasant. There is therefore a demand for a pharmaceutical formulation containing fluticasone propionate in a form suitable for nebulisation.

The present invention accordingly provides, in a first aspect, a formulation suitable for nebulisation comprising:-
(a) Fluticasone propionate, having a particle size of less than 12µm (microns);
(b) one or more surfactants;
(c) one or more buffer agents; and
(d) water.

Fluticasone propionate may be prepared by methods known in the art, for example, as disclosed in GB 2088877. It will be appreciated that solvates of fluticasone propionate can be prepared and, accordingly, the present invention extends to formulations comprising physiologically acceptable solvates of fluticasone propionate. The particle size of the crystalline material may be reduced by conventional methods, for example, by micronisation, and should be such as to permit inhalation of substantially all the medicament into the lungs upon administration of the nebulised formulation. Suitably the particle size will be in the range of 0.5 to 12µm (microns), such as 1 to 6µm (microns).

For introduction of the fluticasone propionate into the lungs, the droplet size of the nebulised formulation is an important parameter. Droplet size depends to some extent on the type of nebuliser used, whether a facemask or a mouthpiece is used and the pressure or flow rate of the compressed gas, as well as on the physical properties of the formulation for nebulisation. The nebulised formulation will be heterodisperse, i.e. droplets will cover a range of sizes. Typically, mean droplet size will be in the range of 0.5 to 15µm (microns), preferably 0.5 to 10µm (microns), more preferably less than 5µm (microns).

The formulation according to the invention desirably contains 0.5 to 10% w/w, preferably 1 to 9% w/w especially 1.5 to 6.5% w/w, of fluticasone propionate relative to the total weight of the solid ingredients of the formulation.

The surfactants used in the formulations of the present invention must be physiologically acceptable upon administration by inhalation. Within this category are included surfactants such as sorbitan trioleate (Span^{R}85), sorbitan mono-oleate, sorbitan monolaurate, polyoxyethylene (20) sorbitan monolaurate, polyoxyethylene (20) sorbitan mono-oleate, natural lecithin, oleyl polyoxyethylene (2) ether, stearyl polyoxyethylene (2) ether, lauryl polyoxyethylene (4) ether, block copolymers of oxyethylene and oxypropylene, synthetic lecithin, diethylene glycol dioleate, tetrahydrofurfuryl oleate, ethyl oleate, glyceryl mono-oleate, polyethylene glycol 400 and glyceryl monolaurate.

Particularly preferred surfactants of use in the formulations of the present invention are sorbitan monolaurate and polyoxyethylene (20) sorbitan monolaurate (also known as polysorbate 20).

Suitably the formulations according to the invention contain 0.25 to 0.75% w/w, preferably 0.4 to 0.6% w/w, especially 0.45 to 0.55% w/w, of surfactant relative to the total weight of the solid ingredients of the formulation.

Preferably, the formulation according to the invention contains sorbitan monolaurate and polyoxyethylene (20) sorbitan monolaurate in a ratio of 1:7.5 to 1:8.25, such as 1:7.7 to 1:8.1

The formulations according to the invention are buffered to a pH of from about 5 to 7, preferably 6. Suitable buffers are those which are physiologically acceptable upon administration by inhalation. Such buffers include citric acid buffers and phosphate buffers, of which phosphate buffers are preferred. Particularly preferred buffers for use in the formulations of the invention are monosodium phosphate dihydrate and dibasic sodium phosphate anhydrous.

The formulations according to the invention will desirably be isotonic. The formulations may be adjusted to isotonicity by addition of a suitable salt, for example, sodium chloride.

Thus, in a preferred embodiment, the formulations according to the invention additionally comprise sufficient sodium chloride, or another suitable pharmaceutically acceptable salt, to provide an isotonic composition.

In a particularly preferred embodiment, the invention provides a formulation suitable for administration by nebulisation, which formulation consists of:
(a) 0.5 - 2.2mg fluticasone propionate (micronised);
(b) 0.12 - 0.18mg polyoxyethylene (20) sorbitan monolaurate;
(c) 0.015 - 0.025mg sorbitan monolaurate;
(d) 18.5 - 19mg monosodium phosphate dihydrate;
(e) 3.2 - 3.7mg dibasic sodium phosphate anhydrous;
(f) 9.4 - 9.8 mg sodium chloride; and
(g) water for injection to 2.0ml.

Thus, it will be appreciated that formulations according to the preferred embodiment consist of:
(a) 0.25 - 1.1mgml⁻¹ fluticasone propionate (micronised);
(b) 0.06 - 0.09mgml⁻¹ polyoxyethylene (20) sorbitan monolaurate;
(c) 0.0075 - 0.0125mgml⁻¹ sorbitan monolaurate;
(d) 9.25 - 9.5mgml⁻¹ monosodium phosphate dihydrate;
(e) 1.6 - 1.85mgml⁻¹ dibasic sodium phosphate anhydrous;
(f) 4.7 - 4.9mgml⁻¹ sodium chloride; and
(g) water.

The formulations according to the invention form weakly flocculated suspensions on standing but, surprisingly, these suspensions have been found to be easily redispersed by mild agitation to provide suspensions with excellent delivery characteristics suitable for use in conventional nebulisers, even after prolonged storage.

The chemical and physical stability and the pharmaceutical acceptability of the formulations according to the invention may be determined by techniques well known to those skilled in the art. Thus, for example, the chemical stability of the components may be determined by HPLC assay, for example, after prolonged storage of the product.

The particle size distribution of the formulations according to the invention on nebulisation may be measured by conventional techniques, for example by cascade impaction or by the "Twin Impinger" analytical process. As used herein reference to the "Twin Impinger" assay means "Determination of the deposition of the emitted dose in pressurised inhalations using apparatus A" as defined in British Pharmacopaeia 1988, pages A204-207, Appendix XVII C. Such techniques enable the "respirable fraction" of the formulations to be calculated. As used herein reference to "respirable fraction" means the amount of active ingredient collected in the lower impingement chamber per actuation expressed as a percentage of the total amount of active ingredient delivered per actuation using the twin impinger method described above. The formulations according to the invention have been found to have a respirable fraction of 10% or more by weight of the medicament, such as 10% to 50%, for example 15% to 35%.

The formulations according to the invention may be prepared by conventional methods for the preparation of suspension formulations. Typically the fluticasone propionate is contacted with a small amount of surfactant solution so as to "wet" it before addition to the bulk liquid containing the remaining excipients. Constant mixing is essential to maintain a homogeneous suspension. The bulk suspension is sterilised, conveniently by means of thermal sterilisation using steam. Aliquots of the suspension are conveniently filled into sterile containers, for example unit dose containers such as vials or ampoules which are suitably moulded from thermoplastics.

A further aspect of the present invention comprises a method of treating respiratory disorders such as, for example, asthma, which comprises administration by inhalation of an effective amount of a formulation as herein described.

Formulations of the present invention can, thus, be delivered by a nebuliser in which case aliquots of the suspension formulation are desirably filled into sterile containers as described above. Alternatively, the formulations of the present invention can be used as a nasal drop presentation. Thus, aliquots of the suspension formulation are desirably filled into sterile, small volume containers adapted for that delivery route.

The invention is further illustrated by the following non-limiting examples.

| Example 1 | mg |
|---|---|
| Fluticasone propionate (micronised) | 0.525 |
| Polyoxyethylene (20) sorbitan monolaurate | 0.14 |
| Sorbitan monolaurate | 0.018 |
| Monosodium phosphate dihydrate | 18.80 |
| Dibasic sodium phosphate anhydrous | 3.50 |
| Sodium chloride | 9.60 |
| Water for injection to | 2.00ml |

It will be appreciated that the formulation prepared according to Example 1 consists of:
0.26mgml⁻¹ fluticasone propionate (micronised);
0.07mgml⁻¹ polyoxyethylene (20) sorbitan monolaurate;
0.009mgml⁻¹ sorbitan monolaurate;
9.4mgml⁻¹ monosodium phosphate dihydrate;
1.75 mgml⁻¹ dibasic sodium phosphate anhydrous;
4.8mgml⁻¹ sodium chloride; and
water.

The formulation prepared according to Example 1 was filled into a nebuliser. The particle size distribution on nebulisation was measured as percentage of fluticasone propionate in Stage 2 (fine particle fraction) of the Twin Impinger apparatus and as percentage of fluticasone propionate in Stages 2-7 (fine particle fraction) of the cascade impactor apparatus. Values of 18.5% and 18.2% respectively were obtained.

| Example 2 | mg |
|---|---|
| Fluticasone propionate (micronised) | 2.10 |
| Polyoxyethylene (20) sorbitan monolaurate | 0.16 |
| Sorbitan monolaurate | 0.02 |
| Monosodium phosphate dihydrate | 18.80 |
| Dibasic sodium phosphate anhydrous | 3.50 |
| Sodium chloride | 9.60 |
| Water for injection to | 2.00ml |

It will be appreciated that the formulation prepared according to Example 2 consists of:
1.05mgml⁻¹ fluticasone propionate (micronised);
0.08mgml⁻¹ polyoxyethylene (20) sorbitan monolaurate;
0.01mgml⁻¹ sorbitan monolaurate;
9.4mgml⁻¹ monosodium phosphate dihydrate;
1.75mgml⁻¹ dibasic sodium phosphate anhydrous;
4.8mgml⁻¹ sodium chloride; and
water.

The formulation prepared according to Example 2 was filled into a nebuliser. The particle size distribution on nebulisation was measured as for Example 1. Values of 22.1% for the Twin Impinger apparatus test and 21.6% for the cascade impactor apparatus test were obtained.

## Claims

1. A suspension formulation suitable for nebulisation, for delivering to the lungs an effective amount of the formulation, said formulation comprising:
(a) fluticasone propionate having a particle size of less than 12 µm (microns);
(b) one or more surfactants;
(c) one or more buffer agents; and
(d) water.

2. A formulation according to claim 1, wherein the fluticasone propionate has a particle size of 1 to 6 µm (microns).

3. A formulation according to any one of claims 1 or 2, wherein the fluticasone propionate is present in an amount of 0.5 to 10% w/w based on the total weight of the solid ingredients of the formulation.

4. A formulation according to any one of claims 1 to 3 wherein the formulation contains two surfactants.

5. A formulation according to any one of claims 1 to 4, wherein the surfactant is present in an amount of 0.25% to 0.75% w/w of the total weight of the solid ingredients of the formulation.

6. A formulation according to any preceding claim, wherein the surfactants are selected from the group consisting of sorbitan trioleate, sorbitan monooleate, sorbitan monolaurate, polyoxyethylene (20) sorbitan monolaurate, polyoxyethylene (20) sorbitan mono-oleate, natural lecithin, oleyl polyoxyethylene (2) ether, stearyl polyoxyethylene (2) ether, lauryl polyoxyethylene (4) ether, block copolymers of oxyethylene and oxypropylene, synthetic lecithin, diethylene glycol dioleate, tetrahydrofurfuryl oleate, ethyl oleate, glyceryl mono-oleate, polyethylene glycol 400 and glyceryl monolaurate.

7. A formulation according to claim 6, wherein the surfactants are sorbitan monolaurate and polyoxyethylene (20) sorbitan monolaurate.

8. A formulation according to claim 7, wherein the sorbitan monolaurate and polyoxyethylene (20) sorbitan monolaurate are present in a ratio of 1:7.5 to 1:8.25.

9. A formulation according to any preceding claim which is buffered to a pH of from 5 to 7.

10. A formulation according to any preceding claim which is isotonic.

11. A formulation according to any preceding claim, consisting of:
(a) 0.25 - 1.1mgml⁻¹ fluticasone propionate (micronised);
(b) 0.06 - 0.09mgml⁻¹ polyoxyethylene (20) sorbitan monolaurate;
(c) 0.0075 - 0.0125mgml⁻¹ sorbitan monolaurate;
(d) 9.25 - 9.5mgml⁻¹ monosodium phosphate dihydrate;
(e) 1.6 - 1.85mgml⁻¹ dibasic sodium phosphate anhydrous;
(f) 4.7 - 4.9mgml⁻¹ sodium chloride; and
(g) water.

12. A formulation according to claim 11 consisting of:
(a) 0.26mgml⁻¹ fluticasone propionate (micronised);
(b) 0.07mgml⁻¹ polyoxyethylene (20) sorbitan monolaurate;
(c) 0.009mgml⁻¹ sorbitan monolaurate;
(d) 9.4mgml⁻¹ monosodium phosphate dihydrate;
(e) 1.75mgml⁻¹ dibasic sodium phosphate anhydrous;
(f) 4.8mgml⁻¹ sodium chloride; and
(g) water.

13. A formulation according to claim 11 consisting of:
(a) 1.05mgml⁻¹ fluticasone propionate (micronised);
(b) 0.08mgml⁻¹ polyoxyethylene (20) sorbitan monolaurate;
(c) 0.01mgml⁻¹ sorbitan monolaurate;
(d) 9.4mgml⁻¹ monosodium phosphate dihydrate;
(e) 1.75mgml⁻¹ dibasic sodium phosphate anhydrous;
(f) 4.8mgml⁻¹ sodium chloride; and
(g) water.

14. A method of preparing a formulation according to any preceding claim comprising contacting the fluticasone propionate with a solution of surfactant and mixing the resultant drug/surfactant solution with the other components of the formulation.

15. A container comprising a formulation according to any one of claims 1 to 13.

16. A formulation according to any one of claims 1 to 13 which is in the form for use in a nebuliser to produce a plurality of droplets of said formulation, said droplets being suitable for inhalation.

## Patentansprüche

1. Zur Verneblung geeignete Suspensionsformulierung zur Übertragung einer wirksamen Menge der Formulierung in die Lungen, wobei die Formulierung umfaßt:
(a) Fluticasonpropionat mit einer Teilchengröße von weniger als 12 *µ*m (Mikron);
(b) ein oder mehrere Tenside;
(c) ein oder mehrere Pufferstoffe; und
(d) Wasser.

2. Formulierung gemäß Anspruch 1, worin das Fluticasonpropionat eine Teilchengröße von 1 bis 6 *µ*m (Mikron) hat.

3. Formulierung gemäß einem der Ansprüche 1 oder 2, worin das Fluticasonpropionat in einer Menge von 0,5 bis 10 % G/G vorhanden ist, bezogen auf das Gesamtgewicht der festen Bestandteile der Formulierung.

4. Formulierung gemäß einem der Ansprüche 1 bis 3, worin die Formulierung zwei Tenside enthält.

5. Formulierung gemäß einem der Ansprüche 1 bis 4, worin das Tensid in einer Menge von 0,25 bis 0,75 % G/G des Gesamtgewichts der festen Bestandteile der Formulierung vorliegt.

6. Formulierung gemäß einem der vorhergehenden Ansprüche, worin die Tenside ausgewählt sind aus der Gruppe, die aus Sorbitantrioleat, Sorbitanmonooleat, Sorbitanmonolaurat, Polyoxyethylen-(20)-sorbitanmonolaurat, Polyoxyethylen-(20)-sorbitanmonooleat, natürlichem Lecithin, Oleylpolyoxyethylen-(2)-ether, Stearylpolyoxyethylen-(2)-ether, Laurylpolyoxyethylen-(4)-ether, Blockcopolymeren von Oxyethylen und Oxypropylen, synthetischem Lecithin, Diethylenglycoldioleat, Tetrahydrofurfuryloleat, Ethyloleat, Glycerylmonooleat, Polyethylenglycol 400 und Glycerylmonolaurat besteht.

7. Formulierung gemäß Anspruch 6, worin die Tenside Sorbitanmonolaurat und Polyoxyethylen-(20)-sorbitanmonolaurat sind.

8. Formulierung gemäß Anspruch 7, worin das Sorbitanmonolaurat und Polyoxyethylen-(20)-sorbitanmonolaurat in einem Verhältnis von 1:7,5 bis 1:8,25 vorhanden sind.

9. Formulierung gemäß einem der vorhergehenden Ansprüche, die auf einen pH von 5 bis 7 gepuffert ist.

10. Formulierung gemäß einem der vorhergehenden Ansprüche, die isotonisch ist.

11. Formulierung gemäß einem der vorhergehenden Ansprüche, bestehend aus:
(a) 0,25 - 1,1 mg/ml Fluticasonpropionat (mikronisiert);
(b) 0,06 - 0,09 mg/ml Polyoxyethylen-(20)-sorbitanmonolaurat;
(c) 0,0075 - 0,0125 mg/ml Sorbitanmonolaurat;
(d) 9,25 - 9,5 mg/ml Mononatriumphosphatdihydrat;
(e) 1,6 - 1,85 mg/ml zweibasigem wasserfreiem Natriumphosphat;
(f) 4,7 - 4,9 mg/ml Natriumchlorid; und
(g) Wasser.

12. Formulierung gemäß Anspruch 11, bestehend aus:
(a) 0,26 mg/ml Fluticasonpropionat (mikronisiert);
(b) 0,07 mg/ml Polyoxyethylen-(20)-sorbitanmonolaurat;
(c) 0,009 mg/ml Sorbitanmonolaurat;
(d) 9,4 mg/ml Mononatriumphosphatdihydrat;
(e) 1,75 mg/ml zweibasigem wasserfreiem Natriumphosphat;
(f) 4,8 mg/ml Natriumchlorid; und
(g) Wasser.

13. Formulierung gemäß Anspruch 11, bestehend aus:
(a) 1,05 mg/ml Fluticasonpropionat (mikronisiert);
(b) 0,08 mg/ml Polyoxyethylen-(20)-sorbitanmonolaurat;
(c) 0,01 mg/ml Sorbitanmonolaurat;
(d) 9,4 mg/ml Mononatriumphosphatdihydrat;
(e) 1,75 mg/ml zweibasigem wasserfreiem Natriumphosphat;
(f) 4,8 mg/ml Natriumchlorid; und
(g) Wasser.

14. Verfahren zur Herstellung einer Formulierung gemäß einem der vorhergehenden Ansprüche, umfassend das Inkontaktbringen des Fluticasonpropionats mit einer Lösung aus Tensid und Vermischen der resultierenden Arzneistoff/Tensid-Lösung mit den anderen Komponenten der Formulierung.

15. Behälter, umfassend eine Formulierung gemäß einem der Ansprüche 1 bis 13.

16. Formulierung gemäß einem der Ansprüche 1 bis 13, die in der Form zu Verwendung in einem Vernebler zur Erzeugung einer Anzahl von Tröpfchen der Formulierung ist, wobei die Tröpfchen zur Inhalation geeignet sind.

## Revendications

1. Formulation de suspension appropriée pour la nébulisation, pour délivrer aux poumons une quantité efficace de la formulation, ladite formulation comprenant :
(a) du propionate de fluticasone dont la granulométrie est inférieure à 12 µm (microns);
(b) un ou plusieurs agents tensioactifs;
(c) un ou plusieurs agents faisant tampon;
(d) de l'eau.

2. Formulation selon la revendication 1, dans laquelle le propionate de fluticasone possède une granulométrie de 1 à 6 µm (microns).

3. Formulation selon l'une quelconque des revendications 1 ou 2, dans laquelle le propionate de fluticasone est présent en une quantité de 0,5 à 10 % en poids/poids basés sur le poids total des ingrédients solides de la formulation.

4. Formulation selon l'une quelconque des revendications 1 à 3, dans laquelle la formulation contient deux agents tensioactifs.

5. Formulation selon l'une quelconque des revendications 1 à 4, dans laquelle l'agent tensioactif est présent en une quantité de 0,25 % à 0,75 % en poids/poids du poids total des ingrédients solides de la formulation.

6. Formulation selon l'une quelconque des revendications précédentes, dans laquelle les agents tensioactifs sont choisis parmi le groupe constitué par le trioléate de sorbitanne, le monooléate de sorbitanne, le monolaurate de sorbitanne, le monolaurate de sorbitanne de polyoxyéthylène (20), le monooléate de sorbitanne de polyoxyéthylène (20), la lécithine naturelle, l'éther oléylique de polyoxyéthylène (2), l'éther stéarylique de polyoxyéthylène (2), l'éther laurylique de polyoxyéthylène (4), des copolymères séquencés d'oxyéthylène et d'oxypropylène, la lécithine synthétique, le dioléate de diéthylèneglycol, l'oléate de tétrahydrofurfuryle, l'oléate d'éthyle, le monooléate de glycéryle, le polyéthylèneglycol 400 et le monolaurate de glycéryle.

7. Formulation selon la revendication 6, dans laquelle les agents tensioactifs sont le monolaurate de sorbitanne et le monolaurate de sorbitanne de polyoxyéthylène (20).

8. Formulation selon la revendication 7, dans laquelle le monolaurate de sorbitanne et le monolaurate de sorbitanne de polyoxyéthylène (20) sont présents dans le rapport de 1:7,5 à 1:8,25.

9. Formulation selon l'une quelconque des revendications précédentes, qui est tamponnée jusqu'à un pH de 5 à 7.

10. Formulation selon l'une quelconque des revendications précédentes, qui est isotonique.

11. Formulation selon l'une quelconque des revendications précédentes, constituée par:
(a) 0,25 - 1,1 mgml⁻¹ de propionate de fluticasone (micronisé);
(b) 0,06 - 0,09 mgml⁻¹ de monolaurate de sorbitanne de polyoxyéthylène (20);
(c) 0,0075 - 0,0125 mgml⁻¹ de monolaurate de sorbitanne;
(d) 9,25 - 9,5 mgml⁻¹ de phosphate monosodique dihydraté;
(e) 1,6 - 1,85 mgml⁻¹ de phosphate de sodium dibasique anhydre;
(f) 4,7 - 4,9 mgml⁻¹ de chlorure de sodium; et
(g) de l'eau.

12. Formulation selon la revendication 11, constituée par:
(a) 0,26 mgml⁻¹ de propionate de fluticasone (micronisé);
(b) 0,07 mgml⁻¹ de monolaurate de sorbitanne de polyoxyéthylène (20);
(c) 0,009 mgml⁻¹ de monolaurate de sorbitanne;
(d) 9,4 mgml⁻¹ de phosphate monosodique dihydraté;
(e) 1,75 mgml⁻¹ de phosphate de sodium dibasique anhydre;
(f) 4,8 mgml⁻¹ de chlorure de sodium; et
(g) de l'eau.

13. Formulation selon la revendication 11, constituée par:
(a) 1,05 mgml⁻¹ de propionate de fluticasone (micronisé);
(b) 0,08 mgml⁻¹ de monolaurate de sorbitanne de polyoxyéthylène (20);
(c) 0,01 mgml⁻¹ de monolaurate de sorbitanne;
(d) 9,4 mgml⁻¹ de phosphate monosodique dihydraté;
(e) 1,75 mgml⁻¹ de phosphate de sodium dibasique anhydre;
(f) 4,8 mgml⁻¹ de chlorure de sodium; et
(g) de l'eau.

14. Procédé de préparation d'une formulation selon l'une quelconque des revendications précédentes, comprenant la mise en contact du propionate de fluticasone avec une solution d'agent tensioactif et le mélange de la solution résultante de médicament/agent tensioactif avec les autres composants de la formulation..

15. Récipient comprenant une formulation selon l'une quelconque des revendications 1 à 13.

16. Formulation selon l'une quelconque des revendications 1 à 13, qui se présente sous une forme à utiliser dans un nébuliseur pour produire une multitude de gouttelettes de ladite formulation, lesdites gouttelettes étant appropriées pour l'inhalation.
